Europäisches Patentamt

European Patent Office

Office européen des brevets

(19)

(11) Publication number: **0 034 904**
**A2**

(12) **EUROPEAN PATENT APPLICATION**

(21) Application number: **81300635.0**

(22) Date of filing: **17.02.81**

(51) Int. Cl.³: **G 01 N 33/52**

(30) Priority: **20.02.80 US 122912**

(43) Date of publication of application:
**02.09.81 Bulletin 81/35**

(84) Designated Contracting States:
**AT BE CH DE FR GB IT LI LU NL SE**

(71) Applicant: **WARNER-LAMBERT COMPANY**
**201 Tabor Road**
**Morris Plains, New Jersey 07950(US)**

(72) Inventor: **Scheinthal, Bernard M.**
**34 Gathering Road**
**Pine Brook New Jersey 07059(US)**

(72) Inventor: **Chafetz, Lester**
**144 Grant Avenue**
**New Providence New Jersey 07974(US)**

(74) Representative: **Jones, Michael Raymond et al,**
**Haseltine Lake & Co. 28 Southampton Buildings**
**Chancery Lane**
**London WC2A 1AT(GB)**

(54) Process for determining the presence in a liquid of theophylline.

(57) The presence of theophylline in a liquid, such as a body fluid, can be determined, quantitatively or qualitatively, by a colour reaction. The theophylline is converted to theophyllidine by reaction with an alkali such as sodium or potassium hydroxide at an elevated temperature and, after neutralization of the alkali with an organic acid, the theophyllidine formed is coupled with a chromogen to yield a colour whose depth is proportional to the theophylline concentration in the body fluid being tested. Hitherto the requirement for an elevated temperature has required the use of external heat; in the invention there is used a solid alkali which generates heat of solution for the heating step; there may also be used a non-corrosive acid for the neutralization. A tetrazo chromogen yields a bright, relatively stable purple colour. The test can be carried out on, for instance, blood or saliva samples in a simple and effective manner.

-1-

PROCESS FOR DETERMINING THE PRESENCE
IN A LIQUID OF THEOPHYLLINE

This invention relates to a process for determining the presence in a liquid of theophylline; the determination may be qualitative or quantitative, and the liquid may be a sample of a body fluid such as blood or saliva.

Theophylline finds important therapeutic application in the management of an asthmatic patient; it is useful not only as a prophylactic drug but also in the treatment of asthmatic attacks. Studies have revealed that blood levels of theophylline of as little as 5 µg/ml, but generally of 10 to 20 µg/ml, are closely associated with observed therapeutic effects. Thus, patient monitoring and the regular determination of blood levels in the patient is an important adjunct to successful theophylline therapy. By noting theophylline levels in blood, in either blood serum or blood plasma, the physician can determine, firstly, whether the patient under examination is following the prescribed dosage regimen and, secondly, whether this regimen is achieving the necessary level in the blood for the desired therapeutic effect. A quick, accurate and reliable test which can be carried out as a routine office procedure by the physician or a technical assistant and is not dependent upon the use of high technology and sophisticated equipment such as that required for radioimmunoassay or high pressure liquid chromatography would be of wide utility.

As previously disclosed by Truitt et al, in the Journal of Pharmacology and Experimental Therapeutics, 91, (1947), pages 185-189, when theophylline is treated by boiling with a strong aqueous alkali, it yields as the reaction product theophyllidine which can be coupled with an azo compound to yield a coloured product. In particular, using Fast Blue 2B salt as the diazo coupling agent, which salt is a zinc chloride complex of diazotized 5-amino-2-benzoyl-amino-1,4-diethoxy-benzene, and coupling it with the theophyllidine obtained by boiling theophylline with 50% aqueous potassium hydroxide solution, a stable red colour is obtained. The depth of this colour can be utilized to reach a quantitative estimate of initial theophylline concentration.

The procedure for determining blood theophylline levels recommended by Truitt et al involves the haemolysis of the red blood cells and treatment with sodium tungstate to yield a clear liquid which is evaporated to dryness. After a multistage chloroform extraction of the resulting residue to extract the theophylline present and the boiling of the thus extracted theophylline with 50% aqueous KOH as described, the alkali boiling step is followed by neutralization of the alkali present with 50% glacial acetic acid. The mixture obtained is cooled and the theophyllidine formed is coupled with the particular azo compound Fast Blue 2B, i.e. the zinc chloride complex of diazotized 5-amino-2-benzoyl-amino-1,4-diethoxy benzene. The intensity of the red colour which results can be read in a suitable photometer.

Comparison of the absorption observed with a suitably prepared standardized curve relating to absorption at known theophylline concentrations enables the theophylline concentration in the blood sample to be determined. The overall time required for this test procedure is 45 minutes.

According to the present invention, there is provided in a process for determining, quantitatively or qualitatively, the presence of theophylline in a liquid by a colour-forming reaction which comprises reacting any theophylline present in solution in an aqueous medium at an elevated temperature with an alkali hydroxide to convert any theophylline present to theophyllidine and then coupling any theophyllidine formed with an azo chromogen to yield a coloured chromophore, the steps which comprise combining an aqueous solution comprising the liquid to be tested. for theophylline with the alkali hydroxide in solid particulate form whereby the heat of solution of the alkali hydroxide in the aqueous solution provides the heat of reaction to convert any theophylline present to theophyllidine and then coupling any theophyllidine formed while in an acid medium with a tetrazo chromogen.

By means of the present invention the determination of the theophylline concentration in a liquid such as a sample of blood plasma, blood serum or saliva, may be greatly simplified and carried out rapidly and safely with no compromise in the accuracy of the results. The basis of the invention is that, instead of boiling the reaction mixture of theophylline and alkali hydroxide, the necessary heat for the reaction is provided by the heat of solution of the alkali hydroxide in the aqueous solution of theophylline being tested by adding solid pellets or other particles of alkali hydroxide to the aqueous solution or vice versa, and then neutralizing the unreacted alkali hydroxide with a slight excess of a solid organic acid. By then coupling the theophyllidine reaction product in the resulting acid solution with a stable chromogen, for instance a stabilized salt of tetrazotized dianisidine such as Fast Blue B, i.e.

the zinc chloride double salt of tetrazotized o-anisidine, a stable purple colour is obtained whose depth is proportional to the initial theophylline concentration.

When standardized quantities of reactants and reagents are employed the stable purple colour produced may be read on a photometer to give a quantitative result by determining absorbance at a wave length of 540nm. Comparison of this absorbance with a standard curve constructed on the basis of aqueous solutions containing known quantities of theophylline enables the theophylline concentration to be determined on a quantitative basis, as the depth of colour observed and the absorbance is proportional to the concentration of the theophylline in the liquid being tested.

The test with, for instance, saliva requires only about 0.5 ml and is extremely useful in obtaining an immediate qualitative answer to the question of whether or not an asthmatic patient is complying with the theophylline therapy prescribed. The theophylline blood level may be established from the saliva test if the quantitative saliva concentration is first determined and this value is then multiplied by a factor of 2. The ratio of theophylline level in saliva to theophylline level in blood is usually in the region of about 0.48:1 to 0.5:1. However, depending upon the individual, this ratio can vary from 0.3:1 to 0.7:1 with the average being about 0.5:1. Even considering that such individual variations do exist, it can be seen that the test procedure described provides a readily available non-invasive technique for determining fairly accurately and rapidly whether a particular theophylline dosage regimen in a particular patient is providing a therapeutic blood level of theophylline of the order of 5 to 20 µg/ml after this ratio is established.

The use of the alkali hydroxide in solid form to achieve the necessary reaction temperature through the heat of solution of the particles in the aqueous theophylline medium so that the theophylline present will be converted to theophyllidine lends substantially greater convenience and utility to the procedure than that afforded by the prior art. The alkali hydroxide particles can be prepackaged in the appropriate quantity needed for the reaction and the handling and pouring of 50% aqueous caustic solutions of sodium or potassium hydroxide are avoided. Substantial advantages are also obtained by use of a solid organic acid to neutralize the excess alkali hydroxide remaining in the reaction medium after the conversion of the theophylline to theophyllidine. Since the subsequent azo coupling reaction takes place in an acid medium it is very helpful in simplifying the procedure to premeasure and prepackage the organic acid for the neutralization step so that when slowly added to the alkali hydroxide reaction medium all of the base will be neutralized and only a slight excess of acid will remain to provide an acid medium for the azo coupling reaction. These expedients greatly simplify the procedure so that it may then be carried out by relatively inexperienced and untrained office assistants without any loss in the reliability and accuracy of the test procedure.

The accuracy and reliability of the test procedure according to the present invention is substantially enhanced by employing as the chromogen in the coupling reaction the stabilized zinc chloride salt of tetrazotized dianisidine. This tetrazotized compound, also known as Fast Blue B, yields a bright stable purple chromophore when coupled with the theophyllidine present and the resulting depth of colour is an accurate indication of the level of theophyllidine present.

The ease and convenience of the test procedure in determining theophylline blood levels can be greatly enhanced by the use of a small-scale or miniaturized

reverse phase partition chromatography column for isolating the theophylline from other components in a sample of blood serum or blood plasma being tested. While the prior art procedure relies upon a complex separation procedure involving evaportion of the serum followed by several chloroform extractions and a final evaporation, it has been found that by the use of a miniaturized reverse phase partition chromotography column for treating a blood plasma sample a very precise separation of the theophylline present in the plasma can be effected.

A particularly useful miniaturized column for this purpose is one which contains the $C_{18}$ compound octadecylsilane bonded to silica gel which is available in a cartridge form and which may be used as an adjunct or accessory to a hypodermic syringe. These cartridges are available commercially as the $C_{18}$ Sep-Pak cartridge from Water Associates, Inc., Milford, MA 01757, U.S.A.

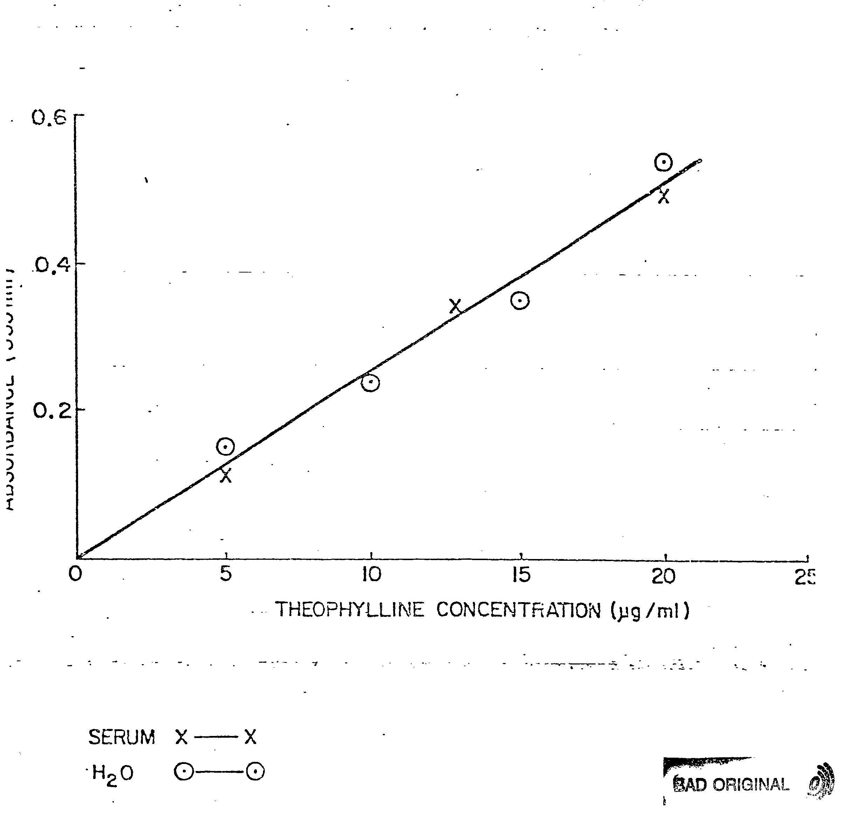

To obtain a quantitative determination of a theophylline blood level using this column chromatography procedure the partition column is prepared by first connecting a cartridge to the end of a Luer tip syringe, then pumping a small amount of methanol through the column followed by a water wash. A measured sample of blood serum or plasma is then pumped through the syringe and the column and the effluent discarded. The theophylline present is retained on the column. A water wash of the column removes all of the soluble blood components and is discarded. The theophylline present is extracted from the column by a methanol wash, the methanol removed by heating and then water and solid particles of sodium hydroxide or potassium hydroxide are added to the aqueous theophylline. After about five minutes the alkali is neutralized with a solid organic acid and the solution cooled. Examples of suitable solid organic acids are citric, tartaric, maleic, malonic, fumaric and succinic acids. Addition to the slightly acid solution of a measured amount of Fast Blue B solution in methanol followed by mixing produces a purple colour as the reaction product whose absorbance is then read after 5 minutes at 535 nm. A substantially linear relationship between absorbance and theophylline concentration exists as shown in the accompanying drawing which represents graphically the relationship between absorbance at 535 nm and theophylline concentration in µg/ml in serum and in water.

In order further to illustrate the present invention the following Example is given.

EXAMPLE

Part I : Preparation of a Fast Blue B Solution:

About 400 mg Fast Blue B salt were shaken with 100 ml methanol for about 15 minutes in a 250 ml stoppered Erlenmeyer flask. The solution was then filtered through S&S No. 508 charcoal-impregnated filter paper, 12.5 cm. The solution should be prepared fresh once a week.

Part II : Qualitative Test for Compliance

To 0.5 ml saliva from a patient on theophylline therapy which had been introduced into an 18 x 150 mm test tube, were added 0.5 ml water and $0.75 \pm 0.05$ g sodium hydroxide pellets. The mixture was shaken vigorously for about 5 minutes. Then cautiously 1.5 g citric acid and 1.0 ml water were added with gentle mixing to avoid a sudden evolution of carbon dioxide. The resulting solution was cooled to room temperature. Then 0.5 ml Fast Blue B reagent was added, mixed well on a vortex mixer, and allowed to stand for 5 minutes. The absorbance at about 540 nm was then read versus a blank and/or compared with a standard.

Part III : Preparation of Chromatography Column

The plunger was removed from a 10 ml Luer tip syringe, and the longer end of a $C_{18}$ Sep-Pak cartridge was connected firmly onto the Luer tip. Five ml methanol were added to the syringe barrel and the plunger used to pump the solvent through the cartridge. The effluent was discarded. The wash step was repeated but this time the wash employed 5 ml of water. With the cartridge connected to the syringe, 0.5 ml of blood serum from a patient on theophylline therapy was added to the syringe and the plunger used to pump the serum through the cartridge. The effluent was discarded. This step was repeated but employing 5 ml of water. The effluent was discarded. Then 2 ml methanol were pipetted into the syringe barrel and the methanol pumped through the cartridge. The first 6 drops were discarded and the balance of the effluent was collected in a 18 mm x 150 mm test tube.

Part IV : The main test

The collected effluent from part III was dried by blowing nitrogen gas over the solution and/or heating with warm water. Then 0.5 ml water was added to the residue and $0.40 \pm 0.05$ g sodium hydroxide pellets. The mixture was shaken for about five minutes. Then 0.8 g citric acid and 1.0 ml water were added carefully and shaken very gently. The reaction product was cooled to room temperature. Then 0.3 ml Fast Blue B solution was added and mixed well on a vibrating mixer such as the Vortex Genie. After about 5 minutes, the absorbance at about 540 nm was read. Fast Blue B reacted quickly with the theophyllidine to form a purple chromophore in 3 to 5 minutes. The maximum absorbance of the dye was between 535 and 540 nm. After about 30 minutes, 25% of the absorbance had become lost, so the reading should be taken within a short time after colour formation.

This above-mentioned reaction is quite specific to theophyllidine. It has been found that solutions containing concentrations even as large as about 1 mg/ml of compounds such as urea, phenobarbital, allantoin, hypoxanthine, xanthine, 1-methylxanthine, 3-methylxanthine, 7-methylxanthine and theobromine do not react with Fast Blue B. Similar concentrations of caffeine, beta-hydroxypropyl theophylline and beta-hydroxyethyltheophylline do react with Fast Blue B to form a coloured product which although initially purple fades rapidly to a yellow colour usually in less than 5 minutes.

The alkali hydroxide employed may be an alkali metal hydroxide or an alkaline earth metal hydroxide.

The percentages referred to hereinbefore are calculated on a weight basis.

CLAIMS

1.     In a process for determining, quantitatively or qualitatively, the presence of theophylline in a liquid by a colour-forming reaction which comprises reacting any theophylline present in solution in an aqueous medium at an elevated temperatute with an alkali hydroxide to convert any theophylline present to theophyllidine and then coupling any theophyllidine formed with an azo chromogen to yield a coloured chromophore, the steps which comprise combining an aqueous solution comprising the liquid to be tested for theophylline with the alkali hydroxide in solid particulate form whereby the heat of solution of the alkali hydroxide in the aqueous solution provides the heat of reaction to convert any theophylline present to theophyllidine and then coupling any theophyllidine formed while in an acid medium with a tetrazo chromogen.

2.     A process according to claim 1, wherein the alkali hydroxide in the medium containing the theophyllidine is neutralized with a slight excess of a solid organic acid prior to the coupling reaction with the tetrazo chromogen.

3.     A process according to claim 2, wherein the alkali hydroxide is particulate sodium hydroxide or potassium hydroxide and the solid organic acid is a dicarboxylic acid.

4.     A process according to claim 2 or 3, wherein the solid organic acid is citric acid.

5.     A process according to any preceding claim, wherein the tetrazo chromogen is a stabilized salt of tetrazotized dianisidine.

6.     A process according to claim 5, wherein the tetrazo chromogen is a stabilized zinc chloride salt of tetrazotized dianisidine.

THEOPHYLLINE CONCENTRATION (μg/ml)

SERUM  X —— X

H₂O  ⊙———⊙

0034904

1/1

BAD ORIGINAL